(19) Europäisches Patentamt / European Patent Office / Office européen des brevets

(11) **EP 3 410 445 B1**

(12) **EUROPEAN PATENT SPECIFICATION**

(45) Date of publication and mention
of the grant of the patent:
**07.05.2025 Bulletin 2025/19**

(21) Application number: **17743903.1**

(22) Date of filing: **10.01.2017**

(51) International Patent Classification (IPC):
**H10N 60/01** (2023.01)     **H10N 60/20** (2023.01)
**G01R 33/3815** (2006.01)

(52) Cooperative Patent Classification (CPC):
**G01R 33/3815; A61B 5/055; H01B 12/04;
H01B 13/00; H01F 6/06; H10N 60/0856;
H10N 60/202**

(86) International application number:
**PCT/JP2017/000370**

(87) International publication number:
**WO 2017/130672 (03.08.2017 Gazette 2017/31)**

(54) **SUPERCONDUCTING WIRE MATERIAL, SUPERCONDUCTING WIRE MATERIAL PRECURSOR, METHOD FOR PRODUCING SUPERCONDUCTING WIRE MATERIAL, SUPERCONDUCTING COIL, MRI AND NMR**

SUPRALEITENDES DRAHTMATERIAL, VORLÄUFER EINES SUPRALEITENDEN DRAHTMATERIALS, VERFAHREN ZUR HERSTELLUNG EINES SUPRALEITENDEN DRAHTMATERIALS, SUPRALEITENDE SPULE, MRT UND NMR

MATÉRIAU DE FIL SUPRACONDUCTEUR, PRÉCURSEUR DE MATÉRIAU DE FIL SUPRACONDUCTEUR, PROCÉDÉ DE PRODUCTION DE MATÉRIAU DE FIL SUPRACONDUCTEUR, BOBINE SUPRACONDUCTRICE, MRI ET NMR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2016 JP 2016013904**

(43) Date of publication of application:
**05.12.2018 Bulletin 2018/49**

(73) Proprietor: **Hitachi, Ltd.**
**Tokyo 100-8280 (JP)**

(72) Inventors:
• **KODAMA Motomune**
  **Tokyo 100-8280 (JP)**
• **TANAKA Hideki**
  **Tokyo 100-8280 (JP)**
• **KOTAKI Hiroshi**
  **Tokyo 100-8280 (JP)**
• **NISHI Kazuya**
  **Tokyo 100-8280 (JP)**
• **SUZUKI Takaaki**
  **Tokyo 100-8280 (JP)**

(74) Representative: **MERH-IP Matias Erny Reichl Hoffmann**
**Patentanwälte PartG mbB**
**Paul-Heyse-Straße 29**
**80336 München (DE)**

(56) References cited:
**EP-A1- 2 843 671      WO-A1-2013/161930
WO-A1-2015/107636    US-A1- 2002 198 111
US-A1- 2004 204 321    US-A1- 2007 123 427**

• **ANONYMOUS: "Hardnesses of the elements (data page): Difference between revisions - Wikipedia", 29 October 2015 (2015-10-29), XP055610839, Retrieved from the Internet <URL:https://en.wikipedia.org/w/index.php? title=Hardnesses_of_the_elements_(data_page) &diff=688147665&oldid=688125678> [retrieved on 20190805]**

## Description

Technical Field

The present invention relates to a superconducting wire having good critical current characteristics.

Background Art

$MgB_2$, as a metal-based superconductor, which has the highest critical temperature of about 40 K, is expected to be applied as a superconducting wire or a superconducting magnet.

A general preparation method of a superconducting wire is a powder-in-tube (PIT method). In this method, a metal tube is filled with base powder, a diameter reduction is performed by a method of drawing, and thereby it is possible to prepare a single-core wire (wire having one superconducting filament) . In addition, when a metal tube is filled with single-core wires and is subjected to the diameter reduction again, a multi-core wire (wire having a plurality of superconducting filaments) is obtained. A method of using $MgB_2$ as the base powder is referred to as an ex situ method. A method of using a mixture of magnesium powder and boron as the base powder is referred to as an in situ method.

When a superconducting wire is used, it is desirable that the superconducting wire has a high critical current density. The critical current density means upper limit current density at which it is possible to perform energization with zero resistance. The critical current density of a superconductor is determined by flux pinning. Lines of magnetic flux are quantized and infiltrate into a (type-II) superconductor, and the Lorentz force acts on the lines of magnetic flux during energization. When the lines of magnetic flux make movement due to the Lorentz force, a loss occurs. Therefore, in order to obtain the high critical current density of the superconductor, it is necessary to introduce defects or inhomogeneities to the superconductor such that the lines of magnetic flux has pinning.

In $MgB_2$, the flux pinning is likely to mainly occur on a crystal grain boundary. In order to increase the flux pinning of the crystal grain boundary, it is effective to reduce crystallinity and to decrease a grain size. $MgB_2$ has high crystallinity at a temperature range of 800°C or above and is likely to achieve grain growth. However, in terms of flux pinning, synthesis at a low temperature of 700°C or below is effective to achieve high critical current density of $MgB_2$ (for example, see NPL

1). On the other hand, a problem arises in that it is difficult to obtain microstructure of $MgB_2$ which is dense to have good continuousness in the PIT method. In the ex situ method, after the diameter reduction, a heat treatment is performed in order to sinter $MgB_2$ powder. At this time, in order to sufficiently bond the powder, it is effective to perform the heat treatment at a high temperature from 800°C to 900°C. However, as described above, it is not preferable to perform the heat treatment at the high temperature in terms of the flux pinning. In the in situ method, after the diameter reduction, a heat treatment for synthesizing $MgB_2$ from magnesium and boron. At this time, even at a heat treatment at a low temperature of 700°C or below, $MgB_2$ having good powder bonding is produced. However, since a reaction of producing $MgB_2$ from magnesium and boron is a volume contraction reaction, many voids are formed, and a problem is pointed out in that a final filling rate of $MgB_2$ is low. Since magnesium is dispersed in a region of boron powder such that $MgB_2$ is produced, in the in situ method, a space, in which magnesium powder is originally present, becomes a void, and characteristic microstructure, in which a large number of tens of micrometers of voids are dispersed, is obtained (For example, see NPL 2).

As described above, the characteristics and problems of the most general in situ method and ex situ method in the PIT method are described; however, various attempts to obtain high critical current density, in addition to the methods described above, have been made. As an example, high-energy mixing of magnesium powder and boron powder is performed by using a planetary ball mill apparatus, and thereby an attempt to increase the reactivity of the powders has been made. Hereinafter, this method will be referred to as a mechanical milling method. In the mechanical milling method, there have been reports that a part of $MgB_2$ is produced by mixing energy even when the heat treatment is not performed, $MgB_2$ synthesized in this manner has a high reactivity, and the high critical current density is obtained (for example, see NPLs 3, 4, 5, and 6 and PTL 1).

Citation List

Patent Literature

PTL 1: Japanese Patent No. 4259806

Non Patent Literature

NPL 1: Supercond. Sci. Technol. 21 (2008) 015008
NPL 2: Jpn. J. Appl. Phys. 51 (2012) 010105
NPL 3: Supercond. Sci. Technol. 22 (2009) 125017
NPL 4: Appl. Phys. Lett. 91 (2007) 082507
NPL 5: Supercond. Sci. Technol. 23 (2010) 065001
NPL 6: Supercond. Sci. Technol. 26 (2013) 025005

Further products of the prior art are described in EP 2 843 671 A1, US 2009 / 0011942 A1, US 2004 / 0204321 A1, WO 2015 / 07636 A1 and US 2002 / 0198111 A1.

## Summary of Invention

### Technical Problem

As described above, the reports that the high critical current density is obtained in the mechanical milling method have been made; however, a shape of tape-shaped wire is used in most of the reports. Although there have been some reports of using a round wire, the critical current density is not at all high. In addition, there has been a report that, with a tape wire obtained by the mechanical milling method, a value of the critical current density significantly changes depending on an angle formed between a tape surface and an external magnetic field (for example, see NPL 5).

In an application to a magnetic resonance imaging (MRI) apparatus in which high magnetic field homogeneity is demanded, it is preferable to use a shape of which a round or angular cross section is highly symmetrical. A tape wire having a high aspect ratio is difficult to secure dimensional accuracy of winding wire. Additionally, it is not highly preferable that a wire having critical current density that is anisotropic to a magnetic field direction impose restriction to coil design. Here, an aspect ratio (width/thickness) of a typical $MgB_2$ tape wire is about 10. In addition, anisotropy of the critical current density of the $MgB_2$ tape wire depends on a temperature and a magnetic field; however, some $MgB_2$ tape wires have the anisotropy of about 10 to 100.

According to the present invention, an object thereof is to provide a wire which has a round or angular shape with good symmetry, in which anisotropy of critical current density is not generated, and which exhibits potential of high critical current density obtained by a mechanical milling.

### Solution to Problem

A superconducting wire according to the present invention includes: a $MgB_2$ filament as defined by claim 1. According to another aspect, the invention also provides a precursor as defined by claim 5.

### Advantageous Effects of Invention

The $MgB_2$ wire of the present invention has a round or angular cross-sectional shape which is used without imposing restriction to design of a superconducting device and exhibits potential of the high critical current density by the mechanical milling method.

### Brief Description of Drawings

[Fig. 1] Fig. 1 is a schematic diagram of dies which are used in drawing.
[Fig. 2] Fig. 2 is a schematic diagram of dies which are used in cassette rolling.
[Fig. 3] Fig. 3 shows magnetic field dependence of critical current density of a prepared single-core wire at 10K.
[Fig. 4] Fig. 4 shows schematic diagrams of longitudinal sections of prepared wires (after heat treatment) in Comparative Example 1 and Example 1.
[Fig. 5] Fig. 5 shows photographs of the longitudinal sections of the prepared wires (after the heat treatment) in Comparative Example 1 and Example 1.
[Fig. 6] Fig. 6 is a schematic diagram for describing definition of a major axis of a void.
[Fig. 7] Fig. 7 shows schematic diagrams of the longitudinal sections of the prepared wires (before the heat treatment) in Comparative Example 1 and Example 1.
[Fig. 8] Fig. 8 shows photographs of the longitudinal sections of the prepared wires (before the heat treatment) in Comparative Example 1 and Example 1.
[Fig. 9] Fig. 9 shows schematic diagrams of Wire-I, Wire-M, and Wire-MR.
[Fig. 10] Fig. 10 shows photographs of Wire-I, Wire-M, and Wire-MR.
[Fig. 11] Fig. 11 shows magnetic field dependence of critical current density of a prepared single-core wire at 10K.
[Fig. 12] Fig. 12 shows a cross section of a prepared multi-core wire in Comparative Example 2.
[Fig. 13] Fig. 13 shows a cross section of a prepared multi-core wire in Example 4.
[Fig. 14] Fig. 14 shows a cross-sectional view of a core wire according to Example 4.
[Fig. 15] Fig. 15 is a view of a configuration of an MRI.
[Fig. 16] Fig. 16 is a perspective view of a vertical magnetic field MRI.

### Description of Embodiments

Hereinafter, embodiments of the present invention will be described with reference to the figures.

In order to achieve the object described above, the present invention prepares $MgB_2$ wire by the following procedure. High-energy mixing of magnesium powder and boron powder is performed by using a planetary ball mill apparatus. At this time, it is preferable that mixing energy is set to the extent that the boron powder is dispersed in the magnesium powder, and production of $MgB_2$ is not caused (to the extent that a production peak of $MgB_2$ is not generated in at least powder X-ray diffraction). A metal tube is filled with mixed powder and is reduced in diameter. At this time, at least some of processing steps, like cassette rolling or groove rolling, employ a processing method, in which a portion of a processing jig which comes into direct contact with the wire is not fixed but rotates. Otherwise, in a case of using a technique like drawing in which a portion of a jig that comes into direct contact with the wire is immovable, a processing material is processed while being heated at a degree of temperature at which $MgB_2$ does not substantially produced.

A precursor prepared in such a method (a state before heat treatment is performed before $MgB_2$ is produced) has the following characteristics. Particles of boron are dispersed in a matrix of magnesium in a portion of the precursor of a $MgB_2$ filament in a longitudinal section of the precursor of the $MgB_2$ wire, and number density of voids each having a major axis of 10 $\mu$m or larger is in a range of 5 to 500 mm$^{-2}$. The void is likely to be oriented to a direction perpendicular to an axial direction of the wire.

In addition, the $MgB_2$ wire has a round or angular cross-sectional shape which is used without imposing restriction to design of the superconducting device and exhibits potential of the high critical current density by the mechanical milling method. An aspect ratio of the $MgB_2$ wire of the embodiment is 3 or lower. An aspect ratio (width/thickness) of a typical $MgB_2$ tape wire is about 10.

In addition, anisotropy of the critical current density is 2 or lower at every temperature magnetic field region. Here, when $\theta$ represents an angle formed between an axis of the $MgB_2$ wire and an external magnetic field, and $Jc(\theta)$ represents dependence of Jc on $\theta$, the anisotropy of the critical current density is defined as a ratio of the maximum value and the minimum value of $Jc(\theta)$.

In addition, the $MgB_2$ wire prepared in this method has the following characteristics. The number density of voids each having the major axis of 10 $\mu$m or larger in the portion of the $MgB_2$ filament is, specifically, in a range of 5 to 500 mm$^{-2}$ in the longitudinal section of the $MgB_2$ wire. The void is likely to be oriented to a direction perpendicular to the axial direction of the wire.

In a case of preparing a multi-core wire, after single-core wires are prepared, a plurality of the single-core wires are embedded in a metal tube, and a diameter reduction is performed. At some of processing steps after the wires are put in, like cassette rolling or groove rolling, employ a processing method, in which a portion of a processing jig which comes into direct contact with the wire is not fixed but rotates. Otherwise, in a case of using a technique like drawing in which a portion of a jig that comes into direct contact with the wire is immovable, a processing material is processed while being heated at a degree of temperature at which $MgB_2$ does not substantially produced. At this time, a sectional configuration, in which iron is disposed around a $MgB_2$ filament, and a material having hardness higher than pure copper is disposed on the outermost circumference, may be employed.

(Comparative Example 1)

Magnesium powder having a grain size of smaller than 200 mesh and purity of 99.8% and boron powder having a grain size of smaller than 250 nm and purity of 98.5% were used as base powders.

The magnesium powder and the boron powder are weighed to have Stoichiometric composition (a mole ratio of 2:1), and two types of mixed powders I and M are prepared. The mixed powder I is obtained by containing base powder together with a stainless steel ball having a diameter of 10 mm in a plastic container and mixing by a ball mill device. The mixed powder M is obtained by containing 7 g of base powder together with 30 zirconia balls each having a diameter of 10 mm in a zirconia container having a volume of 80 ml and mixing in conditions of 400 rpm for six hours by a planetary ball mill device.

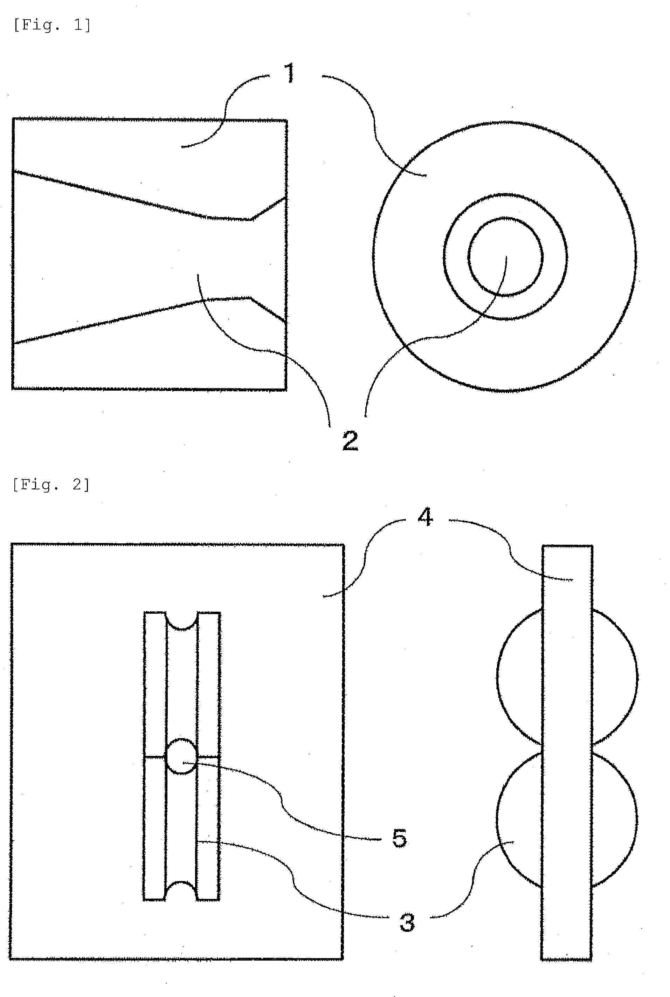

Fig. 1 is a schematic diagram of dies which are used in drawing. An iron tube having an outer diameter of 18 mm and an inner diameter of 13.5 mm was filled with each of the mixed powders, the iron tube was reduced to have a diameter of 0.5 mm by drawing, and a wire (precursor of the $MgB_2$ wire) was prepared. Here, the drawing is a processing method in which the wire is caused to pass through a hole 2 of an empty tapered dies 1 as shown in Fig. 1. The wire is caused to repeatedly pass through the hole 2 of the dies while the dies are replaced with dies having a smaller hole 2 gradually, and thereby it is possible to reduce the diameter of the wire.

Heat treatment was performed in conditions of a temperature of 600°C for three hours in an argon atmosphere, in order to produce $MgB_2$. The wires prepared from both of mixed powders I and M are called Wire-I and Wire-M, respectively.

(Example 1)

The mixed powder M was prepared in the same method as that in Comparative Example 1. An iron tube having an outer diameter of 18 mm and an inner diameter of 13.5 mm was filled with the mixed powder M, the iron tube was reduced to have a diameter of 0.8 mm by drawing. Subsequently, the iron tube was reduced to have a diameter of 0.5 mm by cassette rolling.

Fig. 2 is a schematic diagram of dies which are used in the cassette rolling. As shown in Fig. 2, in the dies 1 of a cassette roll, rolls 3 are attached to a roll fixing instrument 4, and grooves 5 are cut in the rollers. The wire is caused to repeatedly pass through the grooves 5 while the dies are replaced with dies having smaller grooves 5 gradually, and thereby it is possible to reduce the diameter of the wire. The wire receives a force from the fixed dies in the drawing, and the wire receives a force from rotating rolls in the cassette rolling.

Heat treatment was performed in conditions of a temperature of 600°C for three hours in an argon atmosphere, in order to produce $MgB_2$. The prepared wire is called Wire-MR.

The critical current density of the three $MgB_2$ wires prepared in Comparative Example 1 and Example 1 was evaluated. While the temperature of the wires was controlled by spraying of helium gas and heating by a heater, an external magnetic field was applied in a perpendicular direction to an axis of the wire, and current-voltage characteristics were achieved by a DC four-terminal method. A critical current is defined as a current value obtained when a voltage of 1 $\mu$V/cm is generated, and the critical current density is defined as a value obtained by dividing the critical current by a cross-sectional area of a MgB$_2$ filament on a cross section of the wire.

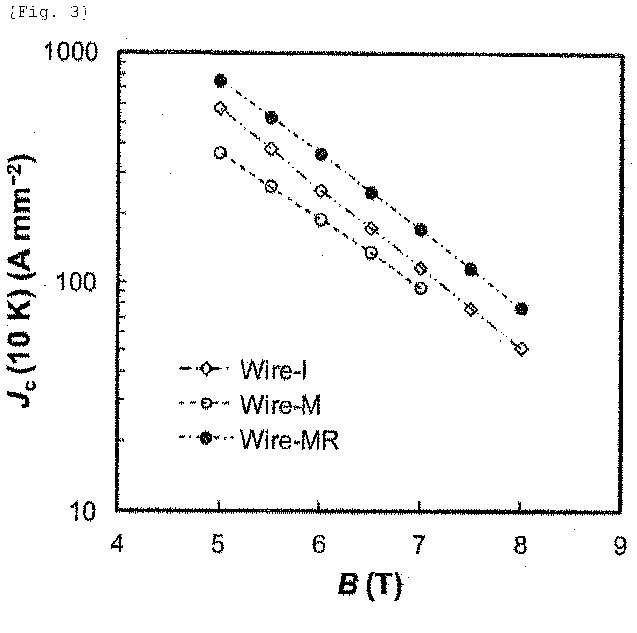

Fig. 3 shows magnetic field dependence of the critical current density of a prepared single-core wire at 10K. Wire-MR had the highest critical current density and, subsequently, the critical current density was high in an order of Wire-I and Wire-M. The result of the above description showed that, by performing a combination of the drawing and the cassette rolling on the mixed powder obtained by the mechanical milling, the high critical current density was obtained.

Microstructure of the three MgB$_2$ wires prepared in Comparative Example 1 and Example 1 was observed. The wire was dried and polished after resin filling, and then a smooth wire-longitudinal-section sample was prepared by cross-section polisher processing (CP processing). The sample was observed with a scanning electron microscope.

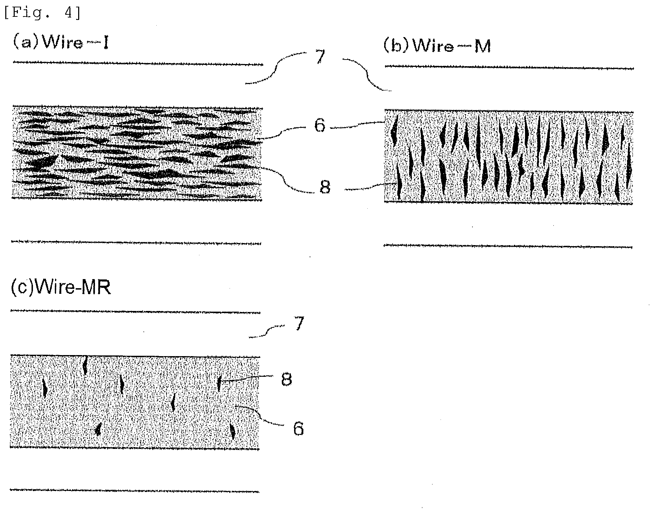

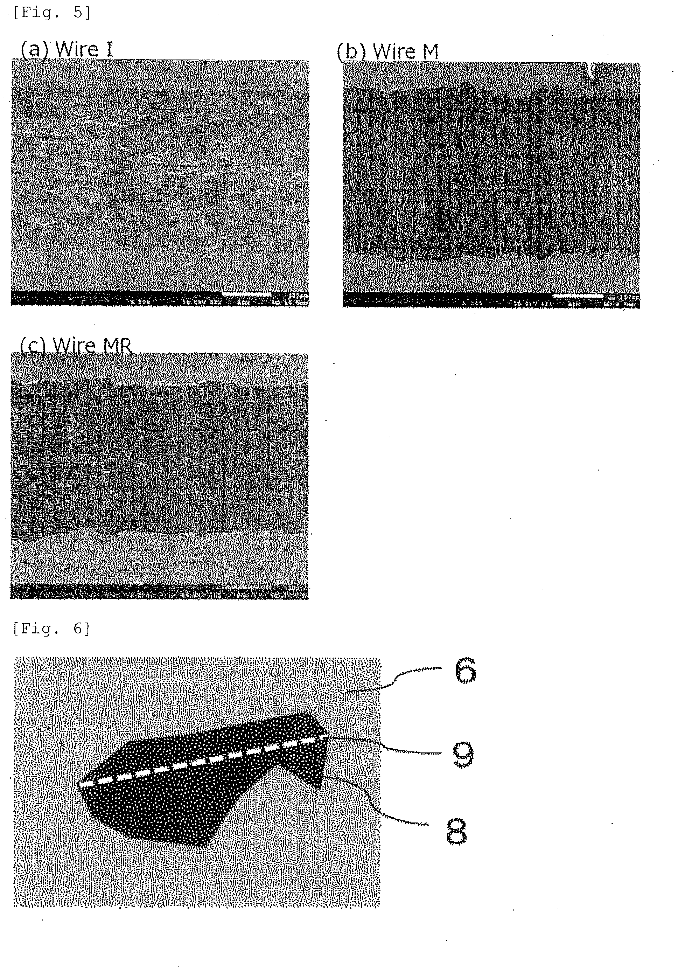

Fig. 4 shows schematic diagrams of longitudinal sections of prepared wires (after the heat treatment) in Comparative Example 1 and Example 1. Fig. 5 shows photographs of the longitudinal sections of the prepared wires (after the heat treatment) in Comparative Example 1 and Example 1. Both of the wires were configured to have a filament 6 in which MgB$_2$ is a main phase and an outer-layer metal member 7 in common, and presence of voids 8 were recognized in the MgB$_2$ filament. On the other hand, the number and directions of voids were remarkably different depending on the wires. In Wire-I, many voids were recognized, and most of the voids were observed to extend in an axial direction of the wire. On the other hand, in Wire-M and Wire-MR, many voids were observed to extend in a direction orthogonal to the axis of the wire. In addition, the number of voids in Wire-M is greater than the number of voids in Wire-MR. For example, the outer-layer metal member 7 is made of iron.

Fig. 6 is a schematic diagram for describing definition of a major axis of a void. A shape of the void 8 in each of the wires was quantified by the following method. The major axis of the void 8 is defined as follows: a line having the longest length of lines connecting two points on an outer circumference of the void on the longitudinal section is defined as the major axis of the void. For example, as in Fig. 6, in a case where the void 7 is present in the filament 6 in which the main phase is MgB$_2$, a white dashed line is the major axis.

Hereinafter, only voids each having the major axis of 10 $\mu$m or larger are targets, and number density n of the voids and an average value $\theta$M of angles $\theta$ ($0° \leq \theta \leq 90°$) formed between the axis of the wire and the major axis of each of the voids are considered. Here, the voids each having the major axis of 10 $\mu$m or larger within a visual field by SEM observation are numbered (i = 1, 2, 3, $\cdots$, and N). When an angle formed between a major axis of an i-th void and the axis of the wire is $\theta_i$, $\theta_M$ is defined by Expression (1).

$$\theta M = \Sigma \theta_i / N \cdots (1)$$

Results of obtaining n and $\theta_M$ of the three wires are shown in Table 1. The values are results of obtaining lengths of the major axes of the voids MgB$_2$ and angles between the axis of the wire and the major axis of each of the voids and averaging the lengths and angles in a plurality of square regions having a length of 300 $\mu$m per side of the MgB$_2$ filament. Wire-I and Wire-M had a high number density of the voids, and Wire-MR had a low number density of the voids. Hence, the high critical current density of Wire-MR is considered to be obtained due to the dense MgB$_2$ filament and an increase in the ratio of a sectional area which can be an effective current path. Wire-I and Wire-M have the same degree of the number density of voids; however, Wire-I has the higher critical current density. This difference is due to a difference of $\theta_M$. In other words, since Wire-M has large $\theta_M$, and thus the voids are oriented to a direction orthogonal to the axis of the wire, in which the voids are more likely to block the current path, a ratio of the sectional area which becomes the effective current path is decreased, and thus the critical current density is considered to be decreased.

[Table 1]

| Sample | n(mm$^{-2}$) | $\theta_M$(deg.) |
|---|---|---|
| Wire-I | $2.1 \times 10^3$ | 16 |
| Wire-M | $1.7 \times 10^3$ | 68 |
| Wire-MR | $0.2 \times 10^3$ | 70 |

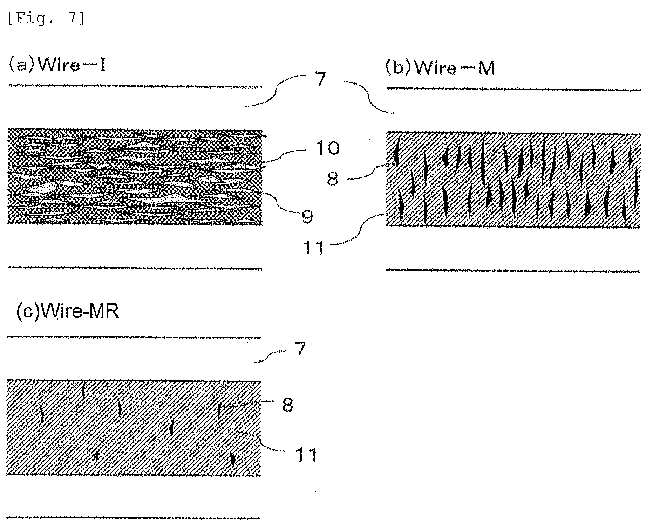

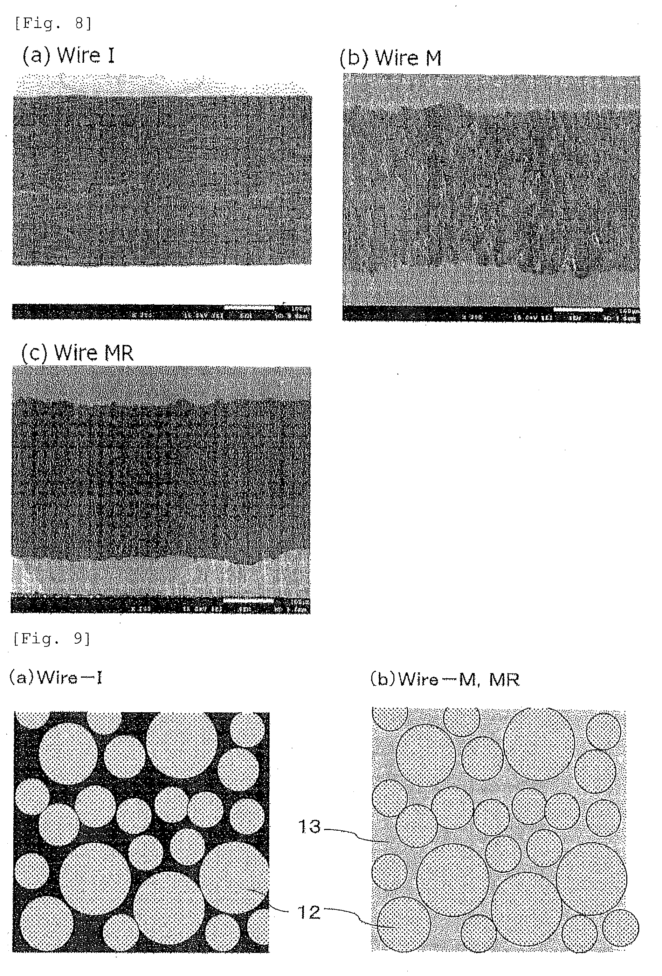

Microstructure on longitudinal sections of the three wires before the heat treatment (that is, precursors of the MgB$_2$ wires) prepared in Comparative Example 1 and Example 2 was observed. Fig. 7 shows schematic diagrams of the longitudinal sections of the prepared wires (before the heat treatment) in Comparative Example 1 and Example 1. Fig. 8 shows photographs of the longitudinal sections of the prepared wires (before the heat treatment) in Comparative Example 1 and Example 1. Both of the wires were configured to have the filament 6 in which magnesium and boron are main phases and the outer-layer metal member 7. In Wire-I, a region 10 filled with boron particles is present in a gap between magnesium particles 9 elongated in the axial direction of the wire. A size and a distribution of magnesium particles 9 are substantially equal to those of the voids 8 after the heat treatment. This is because magnesium is diffused into the regions 10 filled with the boron particles and MgB$_2$ is produced in Wire-I, and the portions, in which magnesium particles are originally present, become the voids 8. The filament 6 of Wire-M was configured to have a uniform structure 11 and the voids 8 in which regions filled with magnesium particles and boron were not distinguished. It was found that there was no significant change in distribution of the voids 8 before and after the heat treatment, and the voids 8 in Wire-M after the heat treatment remained as the voids 8 formed during the processing of the wire. Similar to Wire-M, the filament of Wire-MR was configured to have the uniform structure 11 and the voids 8 in which regions filled with magnesium particles and boron were not distinguished. There was no significant change in the distribution of the voids before and after the heat treatment. Hence, the number density of the voids in Wire-MR is decreased after the heat treatment, because the number density of the voids formed during the processing of the wire is small.

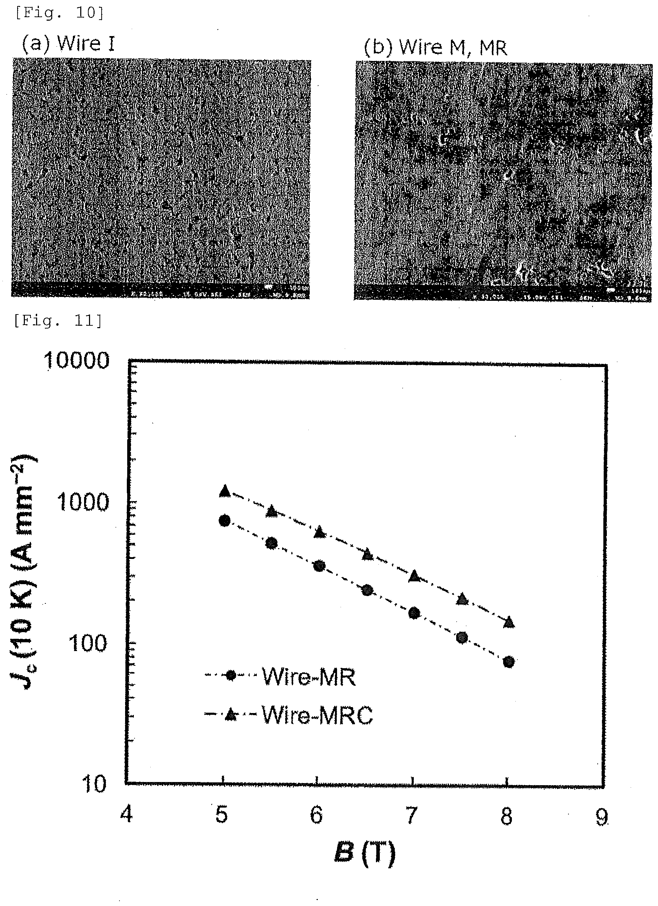

Fig. 9 shows schematic diagrams of Wire-I, Wire-M, and Wire-MR. On the left, an enlarged schematic diagram of the region 10 filled with boron particles in (a) Wire-I is shown. On the right, an enlarged schematic diagram of the uniform structure 11 in (b) Wire-M and Wire-MR is shown. In Wire-M, filling of boron particles 12 having a grain size of about 100 nm is performed, and gaps between boron particles 12 were present. Since the boron particles are hard and do not deform, gaps always remain in the regions filled with boron particles. On the other hand, when a portion viewed to have the uniform structure 11 in Wire-M and Wire-MR was enlarged, it was found that a structure, in which the gaps between boron particles 12 were filled with magnesium 13, is formed. Fig. 10 shows photographs of Wire-I, Wire-M, and Wire-MR.

An observation result of the microstructure suggests that following phenomenon occurs in a process of preparing the wire. A mixing method of base powders influences the distribution of magnesium and boron. In the ball mill mixing of Wire-I, the base particles are mixed as the particles maintain their original shapes. In the drawing, the magnesium particles are subjected to plastic deformation and are elongated in the axial direction of the wire. On the other hand, since the boron particles are hard, the filling rate thereof is increased while the boron particles are repeatedly redisposed without being deformed; however, gaps between boron particles remain until the end. When the heat treatment for synthesizing $MgB_2$ is performed, magnesium is diffused into the regions filled with the boron particles, and $MgB_2$ is produced. However, such a reaction is a volume contraction reaction, and thus the regions, where magnesium is present, remains as voids. When high-energy mixing is performed by using planetary ball milling like Wire-M, the boron particles are kneaded with the magnesium particles in the process of mixing, and powder having a structure in which magnesium as a matrix contains boron is obtained. Powder filled portions are elongated by the drawing; however, plastic deformability of the magnesium particles containing the boron is degraded. Therefore, many voids oriented to the direction perpendicular to the axial direction of the wire are formed without sufficient elongation in the process of the drawing, and thus a filament having bad continuousness is obtained. The voids remain even after the heat treatment and results in a decrease in critical current density. The number density of the voids is very low in Wire-MR, and this suggests that the cassette rolling is effective to block the voids.

The conditions of a planetary ball mill process for preparing the mixed powder are described in detail above; however, what is important is that powder having a structure, in which magnesium as the matrix contains boron, is used. When the powder having the structure is obtained, regardless of conditions of the planetary ball mill process, a method other than the planetary ball mill process may be used. In the planetary ball mill process, the smaller the number of rotations, the smaller the revolution radius, the lower the ratio of total weight of balls to total weight of powder, the larger the inner diameter of the ball, and the shorter the processing time, the more the magnesium and the boron are mixed as maintaining the original shapes. On the other hand, in the planetary ball mill process, when the number of rotations is too large, the revolution radius is too large, the ratio of total weight of balls to total weight of powder is too high, the inner diameter of the ball is too small, and the processing time is too long, the magnesium and the boron react with each other, and $MgB_2$ is produced. Hence, such parameters need to be set within an appropriate range in the planetary ball mill process. NPL 6 discloses a relationship between the conditions of the planetary ball mill process and the mixing energy and discloses that the mixing is performed with energy of 108 J/kg per unit weight of powder, thereby producing a phase of $MgB_2$ during the mixing, and such powder is used, thereby increasing the critical current density of the $MgB_2$ wire. However, the present invention significantly differs from NPL 6 in that the mixing energy in the present invention is lower than 107 J/kg, and thus the mixing is performed in a range in which the production of $MgB_2$ does not occur. In addition, it is checked that production of $MgB_2$ does not actually occur in the powder after the substantial mixing.

Here, the process is referred to as the cassette rolling; however, the process may be referred to as various ways such as a roll forming process, roll forming, cold roll forming, and cold rolling. All of the processes are performed by causing a target object to pass between a plurality of rotating processing jigs and performing deformation processing (diameter reduction). Compared to the drawing in which the wire receives the force from the fixed dies, the cassette rolling is considered to be effective in that a small frictional force is generated between the target object and the dies, damage to filling powder is small because the target object is elongated while the center of the target object is compressed, and the powder is better compressed to be densified.

(Example 2)

The high critical current density is realized by adding the cassette rolling to the wire subjected to the mechanical milling method; however, the characteristics of the microstructure are that the number of coarse void is small (n is small) and the coarse voids are oriented to a direction perpendicular to a longitudinal direction of the wire ($\theta_M$ is large).

In Example 1, the outer diameter was reduced from 18.0 mm to 0.8 mm in the drawing, and the outer diameter was reduced from 0.8 mm to 0.5 mm in the cassette rolling. In other words, the processing method was switched during the reduction at the diameter of 0.8 mm, and thereby it was checked that n is small. In the example, the outer diameter, where the processing method was switched, was changed. As a result, when the outer diameter, where the processing method was switched, was larger, it was observed that n tend to be smaller. However, even in a case where the entire process is configured of the cassette rolling, a value of n is 5 $mm^{-2}$. In addition, in a condition in which n exceeds 500 $mm^{-2}$, the critical current density is not substantially changed from that of Wire-I, and thus employment of the mechanical milling method is not highly superior. In addition, regarding the value of $\theta_M$, no systematic change is observed with respect to the outer diameter where the processing method is switched, and an evaluated sample at this time satisfies $\theta_M \geq 50°$.

Conditions of the number density and shapes of voids are described. Wire-I, Wire-M, and Wire-MR were representative examples; however, as a result of preparing the wires a plurality of times, it was found that the wires prepared under the same conditions has unique characteristics. In both of a case where the powder is mixed by the ball mill device and a case where the powder is mixed by the planetary ball mill device (mechanical milling), the number density of voids is within a numerical value of about $1.0 \times 10^3$ $mm^{-2}$ or higher. In the case where the powder is mixed by the planetary ball mill device, the number density of voids varies within a range of 5 to 500 $mm^{-2}$ through a step of reducing the diameter of the metal tube by further causing the metal tube filled with the mixed powder to pass between the plurality of rotating processing jigs.

In the case where the powder is mixed by the ball mill device, an average value of angles (defined from 0 to 90 degrees) formed between the major axis of each of the voids and the axis of the wire (or the precursor) is smaller than 50 degrees. In the case where the powder is mixed by the planetary ball mill device (mechanical milling), the average value of the angles is 50 degrees or larger.

As described above, a difference in directions of the voids is due to a difference in mechanism of forming the voids. In the case where the powder is mixed by the ball mill device, the energy that is applied to the powder is low, and thus the mixing is performed as magnesium and boron maintain their original shapes. When the metal tube is filled with the mixed powder and is subjected to the drawing, the soft magnesium is stretched and elongated in the longitudinal direction of the wire. Since the magnesium is diffused into the regions of the boron powder by the heat treatment, and $MgB_2$ is produced, the regions in which the magnesium is originally present is changed to voids. The voids reflect the original shape of the magnesium and has a shape extending in the longitudinal direction of the wire, and an angle formed between the major axis of the void and the wire is small. On the other hand, in the case where the powder is mixed by the planetary ball mill device, the energy that is applied to the powder is high, and thus boron particles are kneaded in magnesium. Particles obtained by dispersing boron into a parent phase of magnesium is harder than pure magnesium particles and are difficult to perform plastic deformation. When the process is performed by filling the metal tube with the mixed powder, gaps are likely to be formed between powders adjacent in the longitudinal direction of the wire. In particular, since a force of a tensile component in the longitudinal direction of the wire is strong in the drawing, the voids between the powders are remarkably formed. Since the process proceeds such that the powder is stretched in the longitudinal direction while being crushed in a radial direction of the wire in the cassette rolling, slight deformation occurs even in hard powder, gaps between powder is filled, and thus the number density of voids is small.

(Example 3)

It has been known that a crystalline boron site is substituted with carbon atoms in $MgB_2$, thereby shortening a mean free path of electrons, and the critical current density of a high-magnetic field range is improved by improving an upper critical field. In the example, boron carbide powder having a grain size of 50 nm was added. The powder was weighed so as to obtain composition of $Mg + 1.80B + 0.04B_4C$, and a wire was prepared in the same method of preparing Wire-MR.

Fig. 11 shows the magnetic field dependence of the critical current density of the prepared single-core wire at 10K. As shown in Fig. 11, by adding B4C, further improvement in the critical current density was verified. Hence, it was possible to verify the effect of substitution with carbon in a method using both of the mechanical milling method and the cassette rolling.

In Example 3, fine boron carbide powder was added. Here, the added substance is not limited to the boron carbide, the same effect is obtained even when carbon powder or metallic carbide powder is added. In general, substitution efficiency of carbon depends on a grain size or a type of added material; however, since the powder is mixed by applying high energy by the planetary ball mill device in the mechanical milling method, the substitution efficiency tends to be high even with a material of which substitution efficiency is degraded in a common mixing method and is not suitable for the added material.

(Comparative Example 2)

In the comparative example, the mechanical milling method is applied to manufacturing of a multi-core wire.

A copper-iron composite tube having an outer diameter of 18 mm and an inner diameter of 13 mm was prepared. The copper-iron composite tube is formed of copper on an outer side and iron on an inner side thereof. After the composite tube is filled with mixed powder M, the composite tube is subjected to the drawing to obtain the outer diameter of 12 mm. Next, a wire was processed to have a hexagonal cross section having an opposite side length of 10.2 mm through hexagonal dies, and a hexagonal wire was prepared. Eight hexagonal wires were disposed around a hexagonal copper rod having a cross-sectional shape, in which an opposite side length was 10.2 mm. After an outerside of the eight hexagonal wires was covered with a copper tube having an outer diameter of 40 mm and an inner diameter of 32 mm, a copper wire was inserted into a gap, and an embedded member was prepared. This embedded member was reduced to have an outer diameter of 8 mm in the drawing and to have an outer diameter of 1.5 mm in the cassette rolling, and a multi-core wire was prepared. Heat treatment was performed on the multi-core wire in conditions of a temperature of 600°C for three hours in an argon atmosphere, in order to produce $MgB_2$.

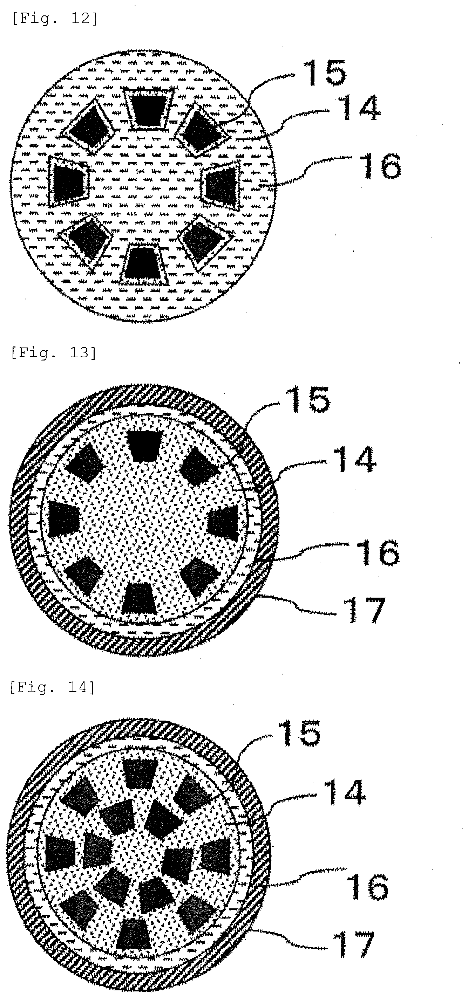

Fig. 12 shows a cross section of the prepared multi-core wire in Comparative Example 2. $MgB_2$ filaments 15 covered with a barrier layer 14 made of iron were disposed in a circle in a stabilizing layer 16 made of copper. As a result of observing details of the sections of the wire, it was recognized that eight $MgB_2$ filaments have a defect in shape, and there were positions at which the iron layer that originally separates the copper from the $MgB_2$ filament is broken. As a result of determining the critical current at a temperature of 10K, the critical current was zero even in a magnetic field 0T. This is because the iron is broken, the mixed powder M and the copper are brought into contact with each other, and the magnesium contained in the mixed powder reacts with the copper, and thereby a lack of magnesium that is required to produce $MgB_2$ occurs.

(Example 4)

In Comparative Example 2, materials of an outer-layer member of the hexagonal wire, the hexagonal rod at the center, and the outer-layer member of the embedded member were changed, and a multi-core wire was prepared. The outer-layer member of the hexagonal wire and the hexagonal rod at the center are made of iron, and the outer-layer member of the embedded member is a Monel-copper composite tube. Here, the Monel-copper composite tube is formed of Monel on an outer side and copper on an inner side thereof.

Fig. 13 shows a cross section of a prepared multi-core wire in Example 4. $MgB_2$ filaments 15 were disposed in a circle in a barrier layer 14 made of iron. The outermost layer 17 made of Monel are separated from the barrier layer 14 made of iron by the stabilizing layer 16 made of copper.

The eight $MgB_2$ filaments had substantially the same shape, no particular breaking was recognized even in the iron on the outer circumference of the $MgB_2$ filament, and it was found that good sectional shapes are obtained.

Deformability of the mixed powder prepared by the mechanical milling method is degraded because boron is contained in magnesium as described above. Therefore, the mixed powder pierces the barrier layer 14 in the process in some cases. Form a result of this time, when all of the $MgB_2$ filaments 15 have a configuration shown in Fig. 12 in which the $MgB_2$ filaments are connected via a barrier member, the configuration is effective to prevent the barrier layer 14 from being broken.

It is important that a material of the barrier layer 14 does not substantially react with the magnesium when the heat treatment for producing $MgB_2$ is performed. In a case of a configuration in which the material that reacts with the magnesium is brought into contact with the $MgB_2$ filament, a lack of magnesium for producing $MgB_2$ occurs. Examples of materials which do not substantially react with magnesium include niobium, tantalum, titanium, and the like, in addition to iron, and the materials may be used as the material for a main component of the barrier layer 14.

In a case where a material of the outermost layer is soft like copper, the $MgB_2$ filament 15 is likely to have a defect in shape, and covering the stabilizing layer 16 with a high-strength material such as Monel as in the example is effective. Nickel, cupronickel, iron, or the like, in addition to Monel, may be used as a material of the outermost layer 17. It is preferable that the material of the outermost layer 17 has Vickers hardness higher than copper.

In the example, the multi-core wire having eight filaments is described; however, it is possible to call a wire having at least two filaments as the superconducting multi-core wire. In addition, the wire may have more than eight filaments. In the superconducting wire, the filament has a small diameter, and thus it is preferable that a large number of filaments are used such that it is possible to avoid a problem of magnetic instability or an AC loss. As an example, Fig. 14 shows a cross section of a 14-core wire.

(Example 5)

In preparing the $MgB_2$ wire using the mixed powder by the mechanical milling method, it is important to add the cassette rolling instead of the drawing. This is because the cassette rolling is effective to improve the filling rate of the mixed powder by the mechanical milling method after the deformability of the mixed powder is degraded. As long as there is a technique in which it is possible to improve the filling rate of the mixed powder by the mechanical milling method, there is no need to be limited to the cassette rolling.

For example, magnesium is hexagonal metal, and thus a sliding surface is limited. Therefore, it is difficult to perform processing on magnesium; however, when magnesium is heated to a temperature of 150°C or above, the processibility is improved. The mixed powder obtained by the mechanical milling method has the structure in which boron is dispersed into the matrix of magnesium. Therefore, when the deformability of the matrix is improved by warm processing, the processibility is improved. Hence, even without using the cassette rolling, it is possible to obtain good microstructure of $MgB_2$ by warm drawing.

However, when a temperature for the warm drawing is too high, brittle $MgB_2$ is produced by the reaction of magnesium and boron, and thus the processibility deteriorates. Therefore, it is preferable that the processing temperature is 500°C at the highest.

(Example 6)

In the example, two methods of preparing a superconducting coil using the superconducting wire of the embodiment are described.

A first method is a wind·and·react method, in which the superconducting wire is wound around a bobbin, and then the heat treatment is added as necessary. In a case of preparing the superconducting coil, it is not possible to increase an excitation speed of the coil when a short circuit occurs between the superconducting wires. Therefore, it is preferable that the superconducting wire is covered with an insulation material. In the wind·and·react method in which the heat treatment is performed in a post-process, a material such as glass material that withstands the heat treatment is used as the insulation material. Then, after the heat treatment is performed, the superconducting wire is fixed by performing resin impregnation as necessary.

A second method is a react·and·wind method, in which the superconducting wire is subjected to the heat treatment, and then the superconducting wire is wound around a bobbin. In this case, it is possible to cover the superconducting wire with the insulation material after the heat treatment, and it is possible to use enamel or the like that does not have heat resistance as the insulation material. After the superconducting wire is wound around the bobbin, the superconducting wire is fixed by performing resin impregnation as necessary.

(Example 7)

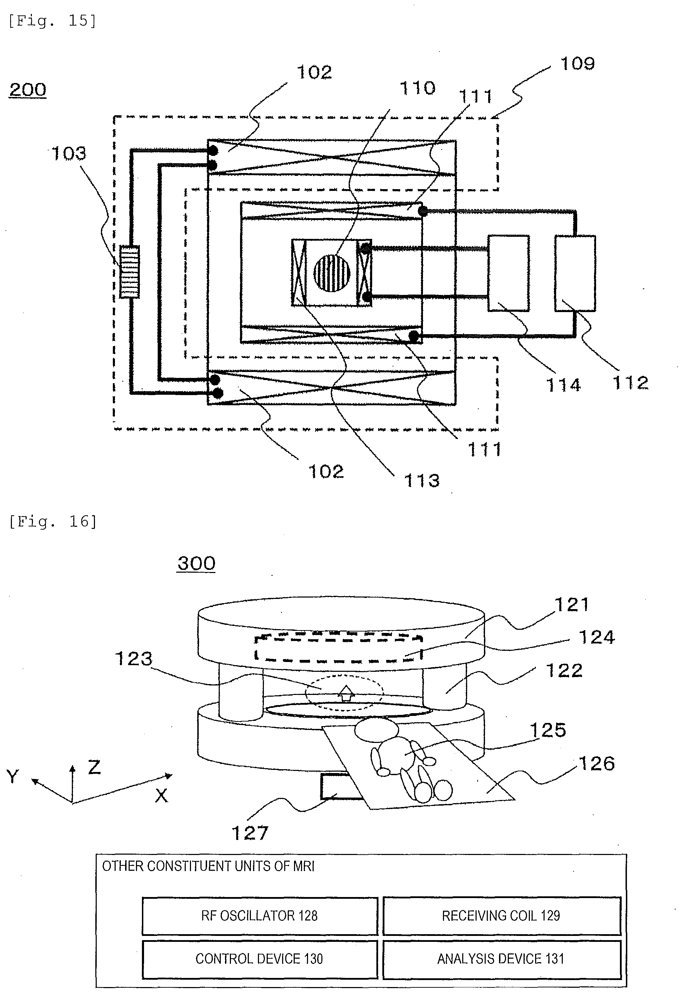

In the example, a configuration of an MRI 200 using the superconducting wire of the embodiment is described. Fig. 15 is a view of a configuration of the MRI.

A superconducting coil 102 using the superconducting wire, along with a persistent current circuit switch 103, is stored in a cryostat 109 and is cooled in a refrigerant or a refrigerator. Persistent current flowing in a circuit that makes the superconducting coil 102 and the persistent current circuit switch 103 generates a magnetostatic field having high time stability at a position of a measurement target. The higher the magnetostatic field intensity, the higher the nuclear magnetic resonance, and the frequency resolution is improved. A time-varying current is supplied to a gradient coil 111 from an amplifier 112 for a gradient magnetic field, and a magnetic field having a spatial distribution is generated at a position of a measurement target 110. Further, an oscillating field having a nuclear magnetic resonance frequency is applied to the measurement target 110 by using a radio frequency (RF) antenna 113 and a RF transmitting/receiving device 114, a resonance signal that is emitted from the measurement target is received, and it is possible to perform tomographic imaging of the measurement target.

(Example 8)

In the example, a vertical magnetic field open MRI using the superconducting wire of the embodiment is described. Fig. 16 is a perspective view of a vertical magnetic field MRI 300.

The MRI includes a pair of magnetostatic field generating units 121 and a connecting member 122, the units and the member are connected such that a central axis Z directing a vertical magnetic field is a rotation target axis. A space formed by the pair of magnetostatic field generating units and the connecting member is referred to as an imaging region 123. A gradient magnetic field generating portion is present in a state in which the imaging region is narrowed. In addition, the MRI includes a bed 126 on which the measurement target is placed and a transport mechanism 127 that transmits a measurement target 125 to the imaging region. Further, the MRI includes a RF oscillator 128 that irradiates the imaging region with an electromagnetic wave having resonance frequency which cause a nuclear magnetic resonance phenomenon to the measurement target, a receiving coil 129 that receives a nuclear magnetic resonance signal, a control device 130 that controls units of the MRI, and an analysis device 131 that analyzes the signal, as the other constituent units not shown in the perspective view.

The pair of magnetostatic field generating units each includes the superconducting coil and generates a uniform magnetostatic field in the imaging region by the superconducting coil. The gradient magnetic field generating portion superimposes gradient magnetic fields in three directions which are orthogonal to the imaging region by any case of switching, on the uniform magnetostatic field such that the magnetic field intensity is gradient in the imaging region. The superimposed gradient magnetic fields imparts positional information to the nuclear magnetic resonance signal, the nuclear magnetic resonance phenomenon appears in a region of interest (a slice plane having a thickness of 1 mm in usual) over the imaging region, and an tomographic image of the measurement target is achieved.

Here, an example of the vertical magnetic field open MRI is described; however, the embodiment can be also applied to a horizontal magnetic field type MRI. In addition, the embodiment can be also applied to a nuclear magnetic resonance (NMR) analyzing apparatus.

The $MgB_2$ wire according to the present invention has a high filling rate of $MgB_2$ and the high critical current density. In a case where a superconducting device such as MRI by using the $MgB_2$ wire is prepared, the following effects are achieved. Since it is possible to perform an operation with high current density, it is possible to reduce material costs of the wires, and this results in a cost reduction of the superconducting device. In addition, since it is possible to perform the operation at a higher temperature, it is possible to reduce a cost for cooling such as electricity charges of the refrigerator. Further, there is no need to use liquid helium, thereby making it simple to handle the device, simplifying a liquid helium tank or an insulation structure. Therefore, it is possible to reduce costs of the superconducting device and to expand a space that a subject enters in the MRI.

Reference Signs List

1:     dies
2:     hole
3:     roll
4:     roll fixing jig
5:     groove
6:     filament
7:     metal outer-layer member
8:     void
9:     magnesium particles
10:    region filled with boron particles
11:    uniform structure
12:    boron particles
13:    matrix of magnesium
14:    barrier layer
15:    $MgB_2$ filament
16:    stabilizing layer
17:    outermost layer

**Claims**

1.  A superconducting wire comprising:
    a $MgB_2$ filament (15),

    **characterised in that** a number density of voids (8) each having a major axis of 10 $\mu$m or larger is

5 to 200 mm$^{-2}$ on a longitudinal section of the superconducting wire, and
**in that** an average value of angles formed between the major axis of each of the voids (8) and the longitudinal axis of the superconducting wire is 60 degrees or larger and 70 degrees or smaller.

2.  The superconducting wire according to Claim 1,

    wherein the superconducting wire has a single-core wire structure having the one $MgB_2$ filament (15), and
    wherein the superconducting wire further comprises a metal layer (7) that covers an outer circumference of the $MgB_2$ filament (15).

3.  The superconducting wire according to Claim 2, wherein the metal layer contains iron.

4.  The superconducting wire according to Claim 1,

    wherein the superconducting wire has a multi-core wire structure having a plurality of the $MgB_2$ filaments (15),
    wherein the superconducting wire further comprises

        a barrier layer (14) that covers an outer circumference of each of the $MgB_2$ filaments (15),
        a stabilizing phase that covers an outer circumference of the barrier layer (14), and
        an outermost layer (17) that covers an outer circumference of the stabilizing phase,

    wherein the barrier layer (14) contains iron, niobium, tantalum, or titanium,
    wherein the stabilizing phase contains copper, and
    wherein the outermost layer (17) contains a material having Vickers hardness higher than copper.

5.  A precursor of a superconducting wire, comprising:

    a microstructure filament obtained by dispersing boron particles in a matrix of magnesium; and
    an outer-layer metal member,

        **characterised in that** a number density of voids (8) each having a major axis of 10 $\mu$m or larger is 5 to 200 mm$^{-2}$ on a longitudinal section of the precursor, and
        **in that** an average value of angles formed between the major axis of each of the voids (8) and an longitudinal axis of the precursor is 60 degrees or larger and larger and 70 degrees or smaller.

6. The precursor of a superconducting wire according to Claim 5,
wherein particles of carbon, boron carbide, or metallic carbide are dispersed in the matrix of magnesium.

7. A superconducting coil comprising:
the superconducting wire according to any one of Claims 1 to 4.

8. A magnetic resonance imaging, MRI, apparatus (200; 300) comprising:

the superconducting coil (102) according to Claim 7; and
analysis means for analyzing a nuclear magnetic resonance signal from a subject (110; 125).

9. A nuclear magnetic resonance, NMR, apparatus comprising:

the superconducting coil according to Claim 7; and
analysis means for analyzing a nuclear magnetic resonance signal from a subject.

**Patentansprüche**

1. Supraleitender Draht, der Folgendes umfasst:

ein $MgB_2$-Filament (15),
**dadurch gekennzeichnet, dass** eine Anzahldichte von Leerstellen (8), die jeweils eine Hauptachse von 10 $\mu$m oder größer aufweisen, auf einem Längsschnitt des supraleitenden Drahtes im Bereich von 5 bis 200 mm$^{-2}$ liegt, und dass ein Mittelwert der Winkel, die zwischen den Hauptachsen jeder der Leerstellen (8) und der Längsachse des supraleitenden Drahtes gebildet sind, 60 Grad oder größer und 70 Grad oder kleiner ist.

2. Supraleitender Draht nach Anspruch 1,

wobei der supraleitende Draht eine Einfachkern-Drahtstruktur aufweist, die das eine $MgB_2$-Filament (15) aufweist, und wobei der supraleitende Draht ferner eine Metallschicht umfasst, die einen Außenumfang des $MgB_2$-Filaments (15) bedeckt.

3. Supraleitender Draht nach Anspruch 2, wobei die Metallschicht Eisen enthält.

4. Supraleitender Draht nach Anspruch 1,

wobei der supraleitende Draht eine Mehrfachkern-Drahtstruktur aufweist, die mehrere der $MgB_2$-Filamente (15) aufweist, wobei der supraleitende Draht ferner Folgendes umfasst:

eine Sperrschicht (14), die einen Außenumfang jedes der $MgB_2$-Filamente (15) bedeckt,
eine Stabilisierungsphase, die einen Außenumfang der Sperrschicht (14) bedeckt, und
eine äußerste Schicht (17), die einen Außenumfang der Stabilisierungsphase bedeckt,

wobei die Sperrschicht (14) Eisen, Niob, Tantal oder Titan enthält,
wobei die Stabilisierungsphase Kupfer enthält und
wobei die äußerste Schicht (17) ein Material mit einer Vickers-Härte, die höher als diejenige von Kupfer ist, enthält.

5. Vorläufer eines supraleitenden Drahtes, der Folgendes umfasst:

ein Mikrostrukturfilament, das durch Dispergieren von Bor-Teilchen in einem Grundstoff aus Magnesium erhalten wird; und
ein Außenschicht-Metallelement,
**dadurch gekennzeichnet, dass** eine Anzahldichte von Leerstellen (8), die jeweils eine Hauptachse von 10 $\mu$m oder größer aufweisen, auf einem Längsschnitt des Vorläufers im Bereich von 5 bis 200 mm$^{-2}$ liegt, und dass ein Mittelwert der Winkel, die zwischen den Hauptachsen von jeder der Leerstellen (8) und einer Längsachse des Vorläufers gebildet sind, 60 Grad oder größer und 70 Grad oder kleiner ist.

6. Vorläufer eines supraleitenden Drahtes nach Anspruch 5,
wobei Teilchen von Kohlenstoff, Borcarbid oder metallischem Carbid in dem Grundstoff aus Magnesium dispergiert sind.

7. Supraleitende Spule, die Folgendes umfasst:
den supraleitenden Draht nach einem der Ansprüche 1 bis 4.

8. Magnetresonanzbildgebungs-Vorrichtung, MRI-Vorrichtung, (200; 300), die Folgendes umfasst:

die supraleitende Spule (102) nach Anspruch 7; und
Analysemittel zum Analysieren eines Kernspinresonanzsignals von einem Subjekt (110; 125).

9.  Kernspinresonanzvorrichtung, NMR-Vorrichtung, die Folgendes umfasst:

    die supraleitende Spule nach Anspruch 7; und Analysemittel zum Analysieren eines Kernspinresonanzsignals von einem Subjekt.


**Revendications**

1.  Fil supraconducteur comprenant :

    un filament MgB$_2$ (15),
    **caractérisé en ce qu'**un nombre de densité de vides (8) ayant chacun un axe majeur de 10 $\mu$m ou plus est de 5 à 200 mm$^{-2}$ sur une section longitudinale du fil supraconducteur, et
    **en ce qu'**une valeur moyenne d'angles formés entre l'axe majeur de chacun des vides (8) et l'axe longitudinal du fil supraconducteur est de 60 degrés ou plus et de 70 degrés ou moins.

2.  Fil supraconducteur selon la revendication 1,

    dans lequel le fil supraconducteur a une structure de fil à cœur unique ayant ledit un filament MgB$_2$ (15), et
    dans lequel le fil supraconducteur comprend en outre une couche métallique (7) qui couvre une circonférence extérieure du filament MgB$_2$ (15).

3.  Fil supraconducteur selon la revendication 2, dans lequel la couche métallique contient du fer.

4.  Fil supraconducteur selon la revendication 1,

    dans lequel le fil supraconducteur a une structure de fil multicœur une pluralité de filaments MgB$_2$ (15),
    dans lequel le fil supraconducteur comprend en outre

    une couche barrière (14) qui couvre une circonférence extérieure de chacun des fi- laments MgB$_2$ (15),
    une phase de stabilisation qui couvre une circonférence extérieure de la couche barrière (14), et
    une couche la plus extérieure (17) qui couvre une circonférence extérieure de la phase de stabilisation,

    dans lequel la couche barrière (14) contient du fer, du niobium, du tantale, ou du titane,
    dans lequel la phase de stabilisation contient du cuivre, et

dans laquelle la couche la plus extérieure (17) contient un matériau ayant une dureté Vickers plus élevée que celle du cuivre.

5.  Précurseur d'un fil supraconducteur, comprenant :

    un filament à microstructure obtenu en dispersant des particules de bore dans une matrice de magnésium ; et
    un élément métallique de couche extérieure, **caractérisé en ce qu'**un nombre de densité de vides (8) ayant chacun un axe majeur de 10 $\mu$m ou plus est de 5 à 200 mm$^{-2}$ sur une section longitudinale du précurseur, et
    **en ce qu'**une valeur moyenne d'angles formés entre l'axe majeur de chacun des vides (8) et un axe longitudinal du précurseur est de 60 degrés ou plus et de 70 degrés ou moins.

6.  Précurseur d'un fil supraconducteur selon la revendication 5,
    dans lequel des particules de carbone, de carbure de bore ou de carbure métallique sont dispersées dans la matrice de magnésium.

7.  Bobine supraconductrice comprenant :
    le fil supraconducteur selon l'une quelconque des revendications 1 à 4.

8.  Appareil d'imagerie à résonance magnétique, IRM, (200 ; 300) comprenant :

    la bobine supraconductrice (102) selon la revendication 7 ; et
    un moyen d'analyse destiné à analyser un signal de résonance magnétique nucléaire à partir d'un sujet (110 ; 125).

9.  Appareil à résonance magnétique nucléaire, RMN, comprenant :

    la bobine supraconductrice selon la revendication 7 ; et
    un moyen d'analyse destiné à analyser un signal de résonance magnétique nucléaire à partir d'un sujet.

[Fig. 1]

[Fig. 2]

[Fig. 3]

[Fig. 4]

(a) Wire-I

(b) Wire-M

(c) Wire-MR

[Fig. 5]

(a) Wire I

(b) Wire M

(c) Wire MR

[Fig. 6]

[Fig. 7]

(a)Wire—I

(b)Wire—M

7

10

8

9

11

(c)Wire-MR

7

8

11

[Fig. 8]

(a) Wire I

(b) Wire M

(c) Wire MR

[Fig. 9]

(a) Wire—I

(b) Wire—M, MR

[Fig. 10]

(a) Wire I

(b) Wire M, MR

[Fig. 11]

[Fig. 12]

[Fig. 13]

[Fig. 14]

[Fig. 15]

[Fig. 16]

| OTHER CONSTITUENT UNITS OF MRI | |
|---|---|
| RF OSCILLATOR 128 | RECEIVING COIL 129 |
| CONTROL DEVICE 130 | ANALYSIS DEVICE 131 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4259806 B
- EP 2843671 A1
- US 20090011942 A1
- US 20040204321 A1
- WO 201507636 A1
- US 20020198111 A1

**Non-patent literature cited in the description**

- *Supercond. Sci. Technol.*, 2008, vol. 21, 015008
- *Jpn. J. Appl. Phys.*, 2012, vol. 51, 010105
- *Supercond. Sci. Technol.*, 2009, vol. 22, 125017
- *Appl. Phys. Lett.*, 2007, vol. 91, 082507
- *Supercond. Sci. Technol.*, 2010, vol. 23, 065001
- *Supercond. Sci. Technol.*, 2013, vol. 26, 025005